# EUROPEAN PATENT SPECIFICATION

(11) **EP 3 113 791 B1**
(45) Date of publication and mention of the grant of the patent: **26.12.2018**
(21) Application number: 15759175.1
(22) Date of filing: 06.03.2015
(51) Int. Cl.: A61K 38/28, A61K 9/08, A61K 31/455, A61K 33/30, A61P 3/10

(54) **NOVEL FAST ACTING INSULIN PREPARATIONS**
NEUARTIGE, SCHNELL WIRKENDE INSULINPRÄPARATE
NOUVELLES PRÉPARATIONS D'INSULINE À ACTION RAPIDE

(30) Priority: 07.03.2014 DK 201400125; 26.03.2014 DK 201400171
(43) Date of publication of application: 11.01.2017
(73) Proprietor: Jørgensen, Klavs Holger, 2830 Virum (DK)
(72) Inventor: Jørgensen, Klavs Holger, 2830 Virum (DK)
(74) Representative: Sun, Yiming
(86) International application number: PCT/DK2015/000010
(87) International publication number: WO 2015/131902

(56) References cited:
- WO-A1-2010/149772
- WO-A1-2012/080362
- WO-A1-2013/186138
- WO-A2-2015/128403
- US-A- 5 382 574

## Description

The present invention relates to stable, extra rapid-acting human or monomeric insulin analog preparations for subcutaneous injection.

### Background of the invention

Insulin treatment of diabetic patients comprises the use of rapid-acting, intermediate-acting, long-acting and biphasic insulin preparations. The rapid-acting insulin preparations have undergone a development towards preparations with still earlier onset of action; from acid solutions to neutral solutions of animal insulin, further to neutral solutions of human insulin (e.g. Actrapid® HM, Humulin® Regular and Insuman® Rapid), and finally to neutral solutions of monomeric insulin analogs. Examples of the latter are NovoRapid® based on B28Asp human insulin ("insulin aspart"), Humalog® based on B28LysB29Pro human insulin and Apidra® based on B3LysB29Glu human insulin. The term "monomeric" alludes to the prevailing formation of monomers from aggregates in solutions of the analog and thereby faster absorption after subcutaneous injection, compared to human insulin, due to the construction of the analog molecule.

Rapid-acting insulin preparations are widely used in connection with a multiple subcutaneous injection (basal/bolus) regimen in which an intermediate-acting insulin is injected twice a day or a long-acting insulin once a day, in order to provide a basal level of plasma insulin, while a rapid-acting insulin is injected at a time most suitable for, as far as possible, normalizing the blood sugar after a meal. In order to mimic the quick release of insulin from pancreas in normal persons by a meal, human insulin has to be injected about ½ hour before the meal in diabetic patients, because of the retarded absorption of the insulin. Due to the faster absorption of monomeric insulin, injection can be made closer to the meal, which is more convenient for the patient. Furthermore, the appearance of monomeric insulin in plasma follows to a higher degree the pattern of plasma insulin appearance in healthy persons after a meal, thus providing an overall improved blood glucose control, including a lower rate of hypoglycaemic events. The above-mentioned commercially available monomeric insulin preparations are therefore preferred for use as the rapid-acting components in a basal/bolus regimen today.

However, there is still a need for yet more rapidly acting insulin preparations. From the point of view of the diabetic patients it would be most desirable to minimize the time length from the injection to the insulin effect. This would mean more freedom and a better metabolic control.

Nicotinamide has been shown to accelerate the absorption of subcutaneously injected human or monomeric insulin, *vide* USP 5,382,574, WO/1996/010417 and WO/2010/149772.

### Description of the invention

The preparations of the invention are solutions and characterized in that they (1) contain human insulin or a monomeric human insulin analog, (2) have a lower concentration of insulin-bound zinc than about 1.8 ions per hexamer of insulin, (3) contain nicotinamide, and (4) has a lower conductivity than about 0.2 mSi/cm.

The preferred content of insulin is in the range from about 0.2 mM to about 6 mM, preferably from about 0.4 mM to about 3 mM, more preferred from about 0.5 to about 0.7 mM.

The preferred content of zinc is less than about 1.2, preferably less than about 0.4, more preferred less than about 0.1, most preferred less than about 0.02 insulin-bound zinc ions per hexamer of human insulin or analog. A low content of insulin-bound zinc, or virtual absence of zinc, is mandatory for obtaining the extra high rate of absorption after injection of the preparations according to the invention. This can be the case, if a zinc-binding, non-insulin substance such as EDTA is present.

The preferred content of nicotinamide is in the range from about 10 to about 500 mM, preferably from about 25 to about 400 mM, more preferred from about 100 to about 350 mM, most preferred from about 150 to about 300 mM.

The conductivity is less than about 0.2 mSi/cm at 22 °C.

A non-ionic detergent such as polysorbate 20 (Tween 20) may be added to the preparations in order to improve physical stability. The preferred concentration of non-ionic detergent ranges from about 5 to about 50 ppm. Non-ionic detergents are well known as stabilizers of protein solutions.

The range of pH is from about 6.0 to about 8.0, preferably from about 6.3 to about 7.5.

The preferred preservative is m-cresol, since solutions of insulins with low contents of zinc have a tendency to precipitate with addition of phenol, but not with addition of m-cresol. In cases where the choice of concentrations of the components, required according to the invention, makes the preparations hypotonic, non-ionic excipients must be added in order to make the preparations isotonic.

### Experiments

Ultrapure Milli-Q® water was used for making and storage of the preparations throughout. Nicotinamide in solution was deionized bypassing an anion exchanger (Sep-Pak Accell Plus QMA) on hydroxyl ion form and a cation exchanger (Sep-Pak Accell Plus CM) on hydrogen ion form.

Glassware was used for making and storage of the preparations.

Centrifugation was used for isolation of precipitates.

Conductivity measurements were performed with a CDM 230 instrument (Radiometer). Rotation of samples was performed by a 820 SWELAB mixer.

The final preparations were run through 0.22µm filters,
Reverse phase HPLC on a C4 column (VYDAC 214TP54) was used in the assessment of chemical stability of preparations.

The mobile phase consisted of A: Water, 0.1% v/v trifluoroacetic acid and B: Acetonitrile, 0.07% v/v trifluoroacetic acid. Elution was performed at a rate of 1 ml/min and at 40°C after the following schedule:
0-5 min: isocratic with 20% B
5-7 min: linear gradient to 24% B
7-27 min: isocratic with 24% B
27-33 min: linear gradient to 27% B
33-42 min: isocratic with 27% B
42-48 min: linear gradient to 40% B
48-50 min: linear gradient to 80% B
50-52 min: isocratic with 80% B
52-53 min: linear gradient to 20% B
53-60 min: isocratic with 20% B

Absorbance of eluate was recorded at 250 nm and 276 nm. In the chromatogram, peaks of insulin-like transformation products, e.g. mono-desamidoinsulins, appeared closely in front of and after the main insulin peak. Then followed an interval with no material appearing. The most hydrophobic insulin-related products, e.g. covalently bound insulin dimer and other high molecular weight products, appear in the linear gradient from 27% to 40% B.

The chemical stability of preparations by storage in closed vials for 14 days at 37° C, in relation to stability by storage at 4°C, was assessed by determination of the percentage distribution between areas of the insulin-like peaks, the main insulin peak and the most hydrophobic insulin-related products, for both temperatures of storage. The percentage formation of products by raising the temperature of storage from 4°C to 37°C could then be assessed.

The physical stability of preparations was assessed by rotation at 37° C of 400 µl of preparations in 100 x 10 mm test tubes, closed by rubber stoppers, and inspecting the physical state at different days after start.

### Example I

A sample of Novo Rapid® was deprived of zinc by adjustment of pH to 3.0, followed by twice salting-out of insulin with NaCl, using centrifugation for isolation of precipitates. The salt-cake was suspended in water and pH adjusted to 5.1, followed by centrifugation. The precipitate was suspended in water and the pH adjusted to 6.0, followed by centrifugation. The precipitate was suspended in water followed by centrifugation. The final precipitate was suspended in water and dissolved by adjusting pH to about 7.4. This solution of virtually zinc-free insulin aspart, was then formulated to Prep. **1** and with added salt to Prep. **2:**

| Prep. No | Insulin aspart, Zinc free. mM | m-cresol M | Nicotinamide M | NaCl M | pH | Conductivity mSi/cm at 22° C |
|---|---|---|---|---|---|---|
| **1** | 0.6 | 0.028 | 0.27 | 0 | 7.4 | 0.18 |
| **2** | 0.6 | 0.028 | 0.17 | 0.05 | 7.4 | 5.1 |

### Example II

A sample of NovoRapid, 100 IU/ml, was deprived of zinc by adjustment of pH to 3.0, and addition of Na₂ EDTA in a mole amount tenfold that of zinc in the sample of NovoRapid, followed by adjustment of pH to 5.1 and centrifugation. The precipitate was suspended in 0.01 M NaCl, followed by centrifugation. The precipitate was suspended in 0.01 M NaCl and the pH adjusted to 6.0, followed by centrifugation. The precipitate was suspended in 0.01 M NaCl, followed by centrifugation. The final precipitate was suspended in water and dissolved by adjusting pH to about 7.4. This solution of virtually zinc-free insulin aspart, was then formulated to Prep. **3** and with added salt to Prep. **4:**

| Prep. No | Insulin aspart, zinc free, mM | m-cresol M | Nicotinamide M | NaCl M | pH | Conductivity mSi/cm at 22° C |
|---|---|---|---|---|---|---|
| **3** | 0.6 | 0.028 | 0.27 | 0 | 7.4 | 0.17 |
| **4** | 0.6 | 0.028 | 0.17 | 0.05 | 7.4 | 5.1 |

### Example III

A sample of Actrapid HM, 100 IU/ml, was processed in a similar way as the sample of NovoRapid in Example II in order to obtain a final solution of virtually zinc-free human insulin, at pH 7.4. The solution was then formulated to Prep. **5** and with added salt to Prep. **6:**

| Prep. No | Human insulin zinc free, mM | m-cresol M | Nicotinamide M | NaCl M | pH | Conductivity mSi/cm at 22° C |
|---|---|---|---|---|---|---|
| **5** | 0.6 | 0.028 | 0.27 | 0 | 7.4 | 0.12 |
| **6** | 0.6 | 0.028 | 0.22 | 0.05 | 7.4 | 5.0 |

### Example IV

A sample of Actrapid HM, 100 IU/ml, was deprived of zinc by adjustment of pH to 3.0, and addition of Na₂ EDTA in a mole amount threefold that of zinc in the sample, followed by adjustment of pH to 5.4 and centrifugation. The precipitate was suspended in water and dissolved by adjusting pH to 7.5 with NaOH. One ion of zinc per hexamer of insulin as zinc acetate and NaCl to a total conductivity of 0.4 mSi/cm at 22° C were added.

| Prep. No | Human insulin zinc free, mM | Ions of zinc per hexamer | m-cresol M | Nicotinamide M | pH | Conductivity mSi/cm at 22° C |
|---|---|---|---|---|---|---|
| 7 | 0.6 | 1 | 0.028 | 0.27 | 7.5 | 0.4 |

**Table 1. Chemical stability. Formation of insulin impurities and survival of insulin by 14 days storage of preparations at 37° C.**

| Prep No | Conductivity mSi/cm | Formation of insulin-like products, % | Formation of most hydrophobic products, % | Survival of insulin, % |
|---|---|---|---|---|
| **1** | 0.18 | 1.8 | 0.9 | 97.3 |
| **2** | 5.1 | 2.9 | 1.3 | 95.8 |
| **3** | 0.17 | 3.8 | 0.9 | 95.3 |
| **4** | 5.1 | 4.5 | 2.0 | 93.5 |
| **5** | 0.12 | 4.4 | 0.8 | 94.8 |
| **6** | 5.0 | 4.1 | 1.3 | 94.6 |
| **7** | 0.4 | 1,6 | 1,0 | 97.4 |
| NovoRapid | - | 1.9 | 0.3 | 97.8 |
| Actrapid | - | 2.4 | 0.4 | 97.2 |
| Apidra | | 1.1 | 1.1 | 97.8 |

**Table 2. Physical stability. Formation of precipitates (fibrils) by rotation of preparations at 37° C. Assessment by observing the appearance of the samples in normal light (NL) and in a concentrated beam of light (CBL) at different days of rotation. Preparations 1 and 2 were only observed in normal light. C: clear O: opal T: turbid,**

| Preparation | Appearance in | 1 day of rotation | 2 days of rotation | 3 says of rotation |
|---|---|---|---|---|
| **1** | NL | **C** | **C** | **C** |
| **2** | NL | **T** | **T** | **T** |
| **3** | NL | **C** | | **C** |
| | CBL | **C** | | **O** |
| **4** | NL | **T** | | **T** |
| | CBL | **T** | | **T** |
| **5** | NL | **C** | **C** | **C** |
| | CBL | **C** | **C** | **C** |
| **6** | NL | **O** | **T** | **T** |
| | CBL | **T** | **T** | **T** |
| **7** | NL | **C** | **C** | |
| | CBL | **C** | **C** | |
| NovoRapid | NL | **T** | **T** | **T** |
| | CBL | **T** | **T** | **T** |
| Actrapid | NL | **C** | **C** | **C** |
| | CBL | **C** | **C** | **c** |
| Apidra | NL | **C** | **O** | **T** |
| | CBL | **C** | **T** | **T** |

### Example V

40 mg of human insulin with about 2.2 Zn⁺⁺per hexamer (Novo Nordisk) was dissolved in 8 ml water with HCl added to pH 3. A trace amount of the radioactive isotope Zn-65 and 10 µmοl Na₂EDTA were added to the solution. The mixture was then run through a column of about 100 mg of the anion exchanger QAE Sephadex A-25 equilibrated with 0.05 M NaCl, followed by washing with 2 ml water. All Zn-65 was retained in the column. The pH of the zinc free eluate was adjusted to 7.4 with NaOH. Equal parts of the solution were transferred to each of two Amicon® Ultra centrifugal -10000 Dalton filter units placed in tubes for collection of filtrate. The volumes were reduced seven times by centrifugation allowing salts to pass through the filters.

The retained solutions were mixed with water to the original volumes. The volumes were again reduced 7 times. The retained volumes were pooled and the insulin concentration measured by HPLC. The pool was used for making a preparation with 0.6 mM zink free human insulin, 0.27 M nicotinamide and 0.028 M m-cresol. The conductivity at 22° C was 0.085 mSi/cm. HPLC of the filtrates showed that less than 1% of the total amount of insulin had passed the filters.

### Discussion of the invention

It appears from Table 1, that the experimental preparations 1, 3 and 5 with the lowest conductivities (0.18, 0.17 and 0.12 mSi/cm, resp.) form hydrophobic products to a lower degree (0.9, 0.9 and 0.8 % *versus* 1.3, 2.0 and 1.3 %) than the experimental preparations **2, 4** and 6 with the highest conductivities (5.1, 5.1 and 5.0 mSi/cm). The formation of hydrophobic products in **1**, **3**, **and 5** is within the range (0.3-1.1 %) of formation for NovoRapid (3 Zn⁺⁺ per hexamer), Actrapid (3Zn⁺⁺ per hexamer) and Apidra (zinc-free). The range of formation of the more insulin-like products, (1.8-4.4 %) for **1, 3** and **5** is at a somewhat higher level than the corresponding range (1.1-2.4 %) for NovoRapid , Actrapid and Apidra.

Based on the method chosen for assessment of physical stability, the results in Table 2 show, that the experimental preparations **1**, **3** and **5** with the lowest conductivities are more physically stable than the experimental preparations **2, 4** and **6** with the highest conductivities, and not less physically stable than NovoRapid, Actrapid and Apidra,

These findings are surprising, since preparations containing zinc free insulin are considered unstable, even when the solution is made in a salt free medium of a zinc free insulin material from an insulin supplier. Such material contains salt in a varying degree, that can cause a higher conductivity in the preparations than that of preparations **1, 3** and **5,** and hence a higher degree of instability at elevated temperatures and rotation. The upper limits of conductivity pertaining to this invention are therefore important.

While preparations **1-6** described in Examples I-III are made from the commercial preparations Actrapid and NovoRapid, the preparation described in Example IV is made from dry insulin powder being a technically relevant starting material for large scale production of preparations according to the present invention. Example V also describes ultrafiltration as an alternative method for desalting compared to the precipitation technique described in Examples I-III.

The following considers, how the present invention relates to published patent applications.

The earlier mentioned USP 5,382,574 deals with preparations comprising insulin or an insulin derivative and nicotinamide or nicotinic acid or a salt thereof. Examples 9 and 10 in this Application describe preparations containing nicotinamide and zinc free BlOAsp human insulin, a monomeric analog. In both examples the absorption of analog after subcutaneous injection of the preparation in pigs was found to be considerably faster than that of a reference preparation containing zinc free analog without nicotinamide, thus demonstrating the absorption promoting effect of nicotinamide, which is the essence of USP 5,382,574. Examples 9 and 10 did not reveal the impact of zinc concentration on the absorption rate of the analog in preparations with nicotinamide. Neither the salt content of the applied zinc-free analog, nor the conductivities of the preparation, nor the stability of the preparations were disclosed.

Example 7 in the same application describes a preparation containing nicotinamide and human insulin with 3 Zn⁺⁺per hexamer. The time until half of the insulin had disappeared after subcutaneous injection of the preparation in pigs was found to be 22 % lower than that of a reference preparation with 3 Zn⁺⁺ per hexamer (composed as Actrapid) and without nicotinamide.

Example 4 in the same application describes a preparation containing nicotinamide, zinc free human insulin. The time until half of the insulin had disappeared after subcutaneous injection of the preparation in pigs was found to be about 46 % lower than that of a reference preparation with 3 Zn⁺⁺ per hexamer (composed as Actrapid) without nicotinamide. Neither the salt content of the applied zinc free insulin, nor the conductivities of the preparation, nor the stability of the test preparation was disclosed.

The earlier mentioned WO/1996/010417 deals with preparations containing insulin aspart and nicotinamide. The only example in the Application describes a test preparation containing 0.6 mmol analog, 3 zinc ions/hexamer, 0.26 mM nicotinamide and 3 g/l phenol (pH 7.4) and a reference preparation with nicotinamide substituted by 16 g/l glycerol. In pigs the test preparation revealed a significantly earlier decrease in plasma glucose and a significantly faster absorption than the reference preparation after subcutaneous injection. The time until half of the insulin had disappeared after subcutaneous injection of the preparations in pigs was found to be about 24 % lower than that of a reference preparation. The invention of the present patent Application deviates from that of WO/1996/010417 in that it contains less zinc (<1,8 Zn⁺⁺/hexamer of insulin). Therefore, injected insulin aspart is faster acting when composed according to the present Application, *vide* the discussion above in connection with USP 5,382,574.

The earlier mentioned WO/2010/149772 deals with preparations containing an insulin compound, a nicotinic compound and arginine. All of the described preparations contain 3 Zn++ per hexamer. Thus, the matter of the present Application is not anticipated. The combination of characteristics pertaining to the present invention has not been disclosed before.

### Summary

The present invention reveals that nicotinamide-containing insulin preparations with extra rapid action after subcutaneous injection can be made sufficiently stable when combined with low concentrations of zinc and low conductivities.

## Claims

1. Preparation for injection in humans comprising: (1) a solution of human insulin or a monomeric insulin analog, (2) insulin-bound zinc in concentration of less than about 1.8 ions per hexamer of insulin, (3) nicotinamide, and wherein the conductivity is less than about 0.2 mSi/cm at 22°C.

2. Preparation according to Claim 1, wherein the insulin is human insulin.

3. Preparation according to Claim 1, wherein the insulin analog is B28Asp human insulin.

4. Preparation according to Claim 1, wherein the insulin analog is B28LysB29Pro human insulin.

5. Preparation according to Claim 1, wherein the insulin analog is B3LysB29Glu human insulin.

6. Preparation according to any of the preceding claims, **characterized in that** the concentration of human insulin or insulin analog is in the range from about 0.2 mM to about 6 mM, preferably from about 0.4 mM to about 3 mM, more preferred from about 0.5 to about 0.7 mM.

7. Preparation according to any of the preceding claims, **characterized in that** the preparation contains less than about 1.2, preferably less than about 0.4, more preferred less than about 0.1, most preferred less than about 0.02 insulin-bound zinc ions per hexamer of human insulin or monomeric insulin.

8. Preparation according to any of the preceding claims, **characterized in that** the concentration of nicotinamide is in the range from about 10 to about 500 mM, preferably from about 25 to about 400 mM, more preferred from about 100 to about 350 mmol, most preferred from about 150 to about 300 mM.

9. Preparation according to any of the preceding claims, **characterized in that** it contains a non-ionic detergent, preferably in the concentration range from about 5 to about 50 ppm.

10. Preparation according to any of the preceding claims, **characterized in that** the pH is in the range from about 6.0 to about 8.0, preferably from about 6.3 to about 7.5.

11. Preparation according to any of the preceding claims, **characterized in that** it contains a preservative, preferably m-cresol in the concentration range from about 10 to 40 mM.

12. Injectable preparation according to any of the preceding claims, said preparation being for use as a medicament for treating or preventing diabetes mellitus.

## Patentansprüche

1. Zubereitung zur Injektion bei Menschen, umfassend: (1) eine Lösung von Humaninsulin oder von einem monomeren Insulinanalogon, (2) Insulin-gebundenes Zink in einer Konzentration von weniger als etwa 1,8 Ionen pro Hexamer Insulin, (3) Nicotinamid, und wobei die Leitfähigkeit weniger als etwa 0,2 mSi/cm bei 22°C beträgt.

2. Zubereitung nach Anspruch 1, wobei das Insulin Humaninsulin ist.

3. Zubereitung nach Anspruch 1, wobei das Insulinanalogon B28Asp Humaninsulin ist.

4. Zubereitung nach Anspruch 1, wobei das Insulinanalogon B28LysB29Pro Humaninsulin ist.

5. Zubereitung nach Anspruch 1, wobei das Insulinanalogon B3LysB29Glu Humaninsulin ist.

6. Zubereitung nach einem der vorhergehenden Ansprüche, **dadurch gekennzeichnet, dass** die Konzentration von Humaninsulin oder Insulinanalogon im Bereich von etwa 0,2 mM bis etwa 6 mM, bevorzugt von etwa 0,4 mM bis etwa 3 mM, mehr bevorzugt von etwa 0,5 bis etwa 0,7 mM liegt.

7. Zubereitung nach einem der vorhergehenden Ansprüche, **dadurch gekennzeichnet, dass** die Zubereitung weniger als etwa 1,2, bevorzugt weniger als etwa 0,4, mehr bevorzugt weniger als etwa 0,1, am meisten bevorzugt weniger als etwa 0,02 insulingebundene Zinkionen pro Hexamer von Humaninsulin oder von monomerem Insulin enthält.

8. Zubereitung nach einem der vorhergehenden Ansprüche, **dadurch gekennzeichnet, dass** die Konzentration von Nicotinamid im Bereich von etwa 10 bis etwa 500 mM, bevorzugt von etwa 25 bis etwa 400 mM, mehr bevorzugt von etwa 100 bis etwa 350 mMol, am meisten bevorzugt von etwa 150 bis etwa 300 mM liegt.

9. Zubereitung nach einem der vorhergehenden Ansprüche, **dadurch gekennzeichnet, dass** sie ein nichtionisches Detergens enthält, bevorzugt im Konzentrationsbereich von etwa 5 bis etwa 50 ppm.

10. Zubereitung nach einem der vorhergehenden Ansprüche, **dadurch gekennzeichnet, dass** der pH-Wert im Bereich von etwa 6,0 bis etwa 8,0, bevorzugt von etwa 6,3 bis etwa 7,5 liegt.

11. Zubereitung nach einem der vorhergehenden Ansprüche, **dadurch gekennzeichnet, dass** sie ein Konservierungsmittel, bevorzugt m-Kresol im Konzentrationsbereich von etwa 10 bis 40 mM enthält.

12. Injizierbare Zubereitung nach einem der vorhergehenden Ansprüche, wobei die Zubereitung zur Verwendung als Medikament zur Behandlung oder Vorbeugung von Diabetes mellitus bestimmt ist.

## Revendications

1. Préparation pour injection à des humains comprenant : (1) une solution d'insuline humaine ou un analogue d'insuline monomère, (2) du zinc lié à l'insuline en une concentration inférieure à environ 1,8 ion par hexamère d'insuline, (3) du nicotinamide, et dans laquelle la conductivité est inférieure à environ 0,2 mSi/cm à 22 °C.

2. Préparation selon la revendication 1, dans laquelle l'insuline est l'insuline humaine.

3. Préparation selon la revendication 1, dans laquelle l'analogue d'insuline est la B28Asp-insuline humaine.

4. Préparation selon la revendication 1, dans laquelle l'analogue d'insuline est la B28LysB29Pro-insuline humaine.

5. Préparation selon la revendication 1, dans laquelle l'analogue d'insuline est la B3LysB29Glu-insuline humaine.

6. Préparation selon l'une quelconque des revendications précédentes, **caractérisée en ce que** la concentration en insuline humaine ou en analogue d'insuline se trouve dans la plage d'environ 0,2 mM à environ 6 mM, de préférence d'environ 0,4 mM à environ 3 mM, de manière davantage préférée d'environ 0,5 mM à environ 0,7 mM.

7. Préparation selon l'une quelconque des revendications précédentes, **caractérisée en ce que** la préparation contient moins d'environ 1,2, de préférence moins d'environ 0,4, de manière davantage préférée moins d'environ 0,1, de manière préférée entre toutes moins d'environ 0,02 ion de zinc lié à l'insuline par hexamère d'insuline humaine ou d'insuline monomère.

8. Préparation selon l'une quelconque des revendications précédentes, **caractérisée en ce que** la concentration en nicotinamide se trouve dans la plage d'environ 10 à environ 500 mM, de préférence d'environ 25 à environ 400 mM, de manière davantage préférée d'environ 100 à environ 350 mmol, de manière préférée entre toutes d'environ 150 à environ 300 mM.

9. Préparation selon l'une quelconque des revendications précédentes, **caractérisée en ce qu'**elle contient un détergent non ionique, de préférence dans la plage de concentration d'environ 5 à environ 50 ppm.

10. Préparation selon l'une quelconque des revendications précédentes, **caractérisée en ce que** le pH se trouve dans la plage d'environ 6,0 à environ 8,0, de préférence d'environ 6,3 à environ 7,5.

11. Préparation selon l'une quelconque des revendications précédentes, **caractérisée en ce qu'**elle contient un conservateur, de préférence du m-crésol dans la plage de concentration d'environ 10 à 40 mM.

12. Préparation injectable selon l'une quelconque des revendications précédentes, ladite préparation étant destinée à une utilisation en tant que médicament pour le traitement ou la prévention du diabète sucré.
